# EUROPEAN PATENT SPECIFICATION

(11) **EP 3 302 254 B1**
(45) Date of publication and mention of the grant of the patent: **23.06.2021**
(21) Application number: 16723096.0
(22) Date of filing: 13.05.2016
(51) Int. Cl.: A61B 5/026, A61B 5/00, A61B 5/027

(54) **CATHETER FOR MEASURING THE BLOOD FLOW OF A BODY TISSUE**
KATHETER ZUR MESSUNG DES BLUTFLUSSES EINES KÖRPERGEWEBES
CATHÉTER POUR MESURER LE FLUX SANGUIN D'UN TISSU CORPOREL

(30) Priority: 29.05.2015 US 201514725802
(43) Date of publication of application: 11.04.2018
(73) Proprietor: CARAG AG, 6340 Baar (CH)
(72) Inventor: LIMACHER, Kuno, 6312 Steinhausen (CH); BERNHARD, Jérôme, 8003 Zurich (CH); STEINER, Claudio, 6340 Baar (CH); RÖTHLIN, Cyrill, 6331 Hünenberg (CH); NAPOLETANO, Daniel, 8193 Eglisau (CH); UHR, Daniel, 6300 Zug (CH); SCHENK, Daniel, 8910 Affoltern am Albis (CH)
(74) Representative: Clerc, Natalia
(86) International application number: PCT/EP2016/060828
(87) International publication number: WO 2016/192958

(56) References cited:
- EP-A1- 1 124 609
- EP-A2- 2 337 496
- US-A1- 2013 274 783
- US-A1- 2015 112 172

## Description

### FIELD OF THE INVENTION

The present invention relates to a catheter, a catheter system and a tube for measuring the flow of blood through a body tissue, particularly through a cerebral tissue. The invention furthermore relates to a catheter for cerebral diagnostics and/or therapy.

### BACKGROUND

Various devices for measuring the flow of blood through a body tissue are known from the prior art. To measure the flow of blood through the cerebral tissue of the brain, catheter-type measurement devices are used which carry measurement sensors at their catheter tip. Such catheter probes are inserted into the cerebral tissue though an opening made in the cranium, in order to carry out a measurement of the flow of blood through said tissue. A number of measurement methods are known for this purpose, for example thermal diffusion, ultrasound methods, and near-infrared spectroscopy in association with an indicator.

For example, EP 1 464 276 A1 discloses a measurement device for determining the flow of blood, in which device two octodes are placed on the surface of the head at a distance from each other. One of the octodes is connected to a radiation source which emits radiation with a near-infrared wavelength. Some of the radiation reflected on the cerebral tissue strikes the second octode, such that the intensity can be determined by an evaluation unit. Indocyanine green is used as the indicator, and a beam of light at a wavelength of between 780 and 910 nm is used. In this method of performing a near-infrared spectroscopy measurement, account must be taken of a large number of external influences that have a disadvantageous impact on the measurement of the flow of blood. The beam of light cannot be conveyed directly to the tissue that is to be measured, and instead it first has to pass through the skin, the skull cap, the dura mater, etc., in order to reach the tissue that is to be examined. As a result, the measurement signal is weakened and distorted by absorption and scattering, for example. It is therefore only possible to measure areas of tissue near to the surface of the head. Areas in the interior of the brain, for example near the floor of the ventricle, can be examined only with inadequate precision by this method.

EP 1 504 715 discloses a catheter with a light-emitting optical conductor and a light-receiving optical conductor, the ends of which optical conductors are arranged at a predetermined distance from each other.

Another device for measuring the flow of blood within the cranium is known from US 5,579,774, for example. A catheter probe, inserted into the interior of the brain, comprises a measurement sensor for carrying out laser Doppler flowmetry. The beam of light of a helium-neon laser at 632.8 nm is guided in the axial direction via a light conductor to the measurement area, which lies distally in a continuation of the probe. Some of the incident light is absorbed and reflected by the surrounding tissue and some by the circulating blood. The reflected light is guided through at least one optical fibre to a processing unit. The light reflected from the moving red blood cells undergoes a Doppler shift, from which it is possible to determine the flow rate. The head of the catheter probe has a rounded tip whose diameter widens conically in the distal direction. The blunt tip is pushed through the opening in the cranium and through the subjacent tissue as far as the measurement area lying within the brain. The whole surface of the blunt tip presses against the cerebral tissue and exerts a pressure that can leave behind permanent damage in the brain.

EP 1 124 609 discloses an implant which is implanted in a patient's chest comprising a pace maker and a medical electric lead. The lead comprises a sensor and a tip electrode.

US 2015/112172 describes a catheter with a sensor device for measuring venous and arterial oxygen saturation in the blood of blood-filled anatomical structures.

### SUMMARY

According to one aspect of the invention a device is made available which measures the flow of blood through a body tissue, in particular through deep-lying cerebral tissue, and which is easy and inexpensive to produce and does not distort the measurement.

According to another aspect of the invention a device is made available which measures the flow of blood through a body tissue, in particular through deep-lying cerebral tissue, and which has the narrowest possible configuration and permits insertion of the device with minimal trauma, i.e. with minimal injury, into cerebral tissue.

According to another aspect of the invention a device is made available which measures the flow of blood through a body tissue, in particular through deep-lying cerebral tissue, and which permits insertion of the measurement device with minimal trauma into cerebral tissue.

According to another aspect of the invention a device is made available which measures the flow of blood through a body tissue, in particular through deep-lying cerebral tissue, and which permits reliable measurement of the flow of blood.

According to another aspect of the invention a device is made available which measures the flow of blood through a body tissue, in particular through deep-lying cerebral tissue, and which is transportable along with a patient.

In an example of the catheter, a single optical fibre is present as optical conductor for the light to be emitted and for the reflected light, and a photodetector and a drainage channel for drainage are present.

In another example of the catheter, a single optical fibre is present as optical conductor for the light to be emitted, as are a photodetector in the catheter for the reflected light, and a drainage channel for drainage.

In another preferred embodiment of the catheter according to the invention, the above-described head part is present, as are a pressure sensor and a drainage channel for drainage.

Said drainage channel can additionally or alternatively be used as a guide channel. In a catheter made of a largely flexible material, a stiff wire is pulled through the guide channel, making it easier to insert the catheter into the body tissue.

A device for measuring the flow of blood through a body tissue comprises a catheter with a catheter head for insertion into the interior of a body tissue, an optical conductor inside the catheter, a light source for emitting a beam of light into the body tissue by means of the optical conductor, and a processing unit for determining the rate of blood flow by means of a beam of light reflected from the body tissue. The catheter has a stiff element, for example a middle piece, with a recess, a cutting, an aperture or an indentation which is oriented inwards into the stiff element and is provided laterally with respect to the longitudinal axis of the catheter. The recess or the like can have a round, oval or parabola-shaped inner surface or, in the case of an indentation for example, can be made up of a plurality of walls. The recess or the like has a surface area from which the optical conductor opens out and from which the beam of light thus emerges, and a further surface area which lies opposite the surface area from which the optical conductor opens out and which is oriented at least partially obliquely with respect to the axis of the optical conductor and preferably also obliquely with respect to the longitudinal axis of the catheter. For simplicity, the two surface areas are referred to herein below as the light exit surface or light exit surface area and as the reflection surface or reflection surface area. The optical conductor emerges from the light exit surface in such a way that the beam of light emitted from the light source is directed to the reflection surface and is deflected by the latter into the surrounding body tissue. The beam of light is absorbed and reflected in the body tissue in a manner characteristic of the flow of blood, as a result of which a reflection light beam is formed which is reflected on the reflection surface and coupled into the optical conductor. The reflection light beam is preferably focussed on the reflection surface. The reflection light beam is transmitted through the optical conductor to the processing unit, where the rate of blood flow can be determined from this delivered signal by comparison with the input signal according to the emitted beam of light.

The catheter thus forms a probe or catheter probe for measuring the flow of blood through the body tissue. The catheter preferably has a specially designed catheter head, as is explained further below, as a result of which it is especially suitable for measurements on cerebral tissue, particularly on deep-lying cerebral tissue. The optical conductor can be formed, for example, by a fibre-optic cable or another light conductor, which is routed through the catheter to the stiff element, for example the middle piece, and to the light exit surface of the latter. The reflection surface can itself have a surface roughness that is of sufficient quality, preferably of mirror-like quality, to allow it to reflect the beam of light from the light conductor. Preferably, a reflector, for example a mirror, is arranged on the oblique surface so as to reflect the beam of light into the surrounding body tissue. It is also advantageous if the reflection surface area has a curved shape in order, on the one hand, to reflect the incident beam of light into the tissue in a focussed manner and, on the other hand, to ensure that the light reflected on the tissue is fed into the optical conductor. In the configuration of the recess with its associated surface areas, the distance between the exit point of the emitted beam of light from the optical conductor and the opposite reflection surface is known and is provided such that the optical conductor focuses the emitted beam on the focal point of the reflection surface if the latter has a curved shape. The emitted beam of light is preferably reflected at an angle of 45° into the surrounding tissue. However, other angles of reflection are also conceivable, e.g. in a range of 30°-60°.

The recess is preferably filled or sealed or plugged with a material transparent to light. The outer surface of the filler material is preferably flush with the circumferential surface of the surrounding area, such that a smooth transition is obtained. Epoxy resin, for example, can be used as filler material. By filling the recess, it is possible to avoid air inclusions in the beam path.

It is preferable to use a light source with coherent light, e.g. a laser or a laser diode, which can emit light in the near infrared range between 780 and 910 nm. Light in this wavelength range is able to penetrate biological tissue. It is preferable to specifically use wavelengths that are suitable for a chosen measurement technique. Light with wavelengths of 785 nm, 850 nm and 905 nm is preferably used, which in particular is absorbed and reflected by oxygenated and deoxygenated haemoglobin and the marker substance indocyanine green. It is also advantageous to use a pulsed light beam or pulsed light beams of variable frequency. The parts of the incident beam of light reflected on the tissue are transformed in the processing unit by means of an analog-digital converter into a meaningful signal relating to the time profile of the presence of oxygenated and deoxygenated blood and of the marker substance.

It is also possible, however, to use a light source that emits light over a broad wavelength spectrum, for example a source of white light. The relevant wavelength ranges for measurement of the flow of blood can then be detected by a spectrometer. However, sources of coherent light have the advantage of requiring less energy.

The catheter probe is also suitable for tissue areas lying far within the brain. For this purpose, the distance between the catheter head and the stiff element, for example middle piece, is advantageously variable. A connection element, e.g. in the form of a tube, of greater or lesser length can be used depending on the desired measurement range or the desired depth of penetration into the brain. The catheter head can be inserted into the brain as far as the ventricle floor, for example, which then serves *inter alia* as reference point for locating the stiff element, for example the middle piece. The measurement of the flow of blood can thus be performed directly *in situ* in the areas of body tissue that are of interest.

The device for measuring the flow of blood through a body tissue also preferably comprises a holder which is placed on the surface of the head, over an insertion opening in the cranium, and which holds the catheter probe in a predetermined position. The holder has, for example, a contact surface for placing onto the surface of the head and, protruding from this contact surface, a guide tube for the catheter. The holder can avoid penetration of infectious substances into the brain during a measurement. It acts as a barrier against pathogens and contaminants. The guide tube is preferably arranged perpendicularly on the contact surface, such that it guides the catheter head perpendicularly through the cranium and then holds it perpendicularly in position. In this position, the catheter probe can be turned about its axis without being deflected from its position and damaging surrounding tissue. By turning the probe, the attainable measurement field is greatly enlarged. A measurement can be performed in a 360° sector around the stiff element, for example the middle piece, of the catheter probe.

The catheter probe can also have at least one X-ray marker, which can indicate the position and orientation of the probe in the tissue when an X-ray measurement is being carried out.

If the catheter head and the stiff element (such as the middle piece) are made of metal, their position can be monitored by X-ray methods. In principle, however, other biocompatible materials are also conceivable, in which case the monitoring by X-ray methods can be achieved by opal additives, by electronic components already used in the catheter, or by markers. Moreover, the catheter can be provided with a scale for the depth of penetration into the body tissue and also with an angle scale for indicating the angle position of the stiff element (such as the middle piece). These features permit a precise locating of the measurement area in the brain.

At the same time, the catheter probe can be equipped with additional measurement sensors. For example, a temperature measurement sensor, e.g. a thermistor or a thermocoupler, can be provided in the stiff element (such as the middle piece). The temperature sensor can be arranged in an outer area of the stiff element, e.g. in a channel. Since metal has an excellent heat conduction coefficient, it is possible, at the same time as measuring the flow of blood through the tissue surrounding the stiff element, to also detect the temperature of the tissue. Contact with the tissue is not necessary in this case. However, if the stiff element (such as the middle piece) is made of plastic, contact between measurement sensor and tissue must be ensured. The leads needed for the measurement can be routed through the catheter to the processing unit, in which the temperature signal is received and converted. However, it is also possible to arrange a temperature measurement sensor in a tube area between the tip of the probe and the stiff element (such as the middle piece).

Moreover, the catheter head and the stiff element (such as the middle piece) of the catheter can be provided with a passage or a drainage channel that permits drainage of the surrounding tissue through the catheter. For this purpose, the catheter head, for example, has at least one opening which leads to the surrounding tissue and which is connected to the drainage channel, such that fluid can be aspirated from the tissue through the channel. Arranged near the drainage opening, there is preferably at least one pressure sensor which is designed, on the one hand, to determine the pressure in the surrounding tissue and, on the other hand, to determine the pressure in the drainage channel. In the event of an occlusion and a subsequent change of pressure in the drainage channel, a signal can be output to the processing unit, such that an alarm can be triggered. In principle, it is also possible to deliver liquid to the tissue by way of the drainage opening, i.e. to inject liquid.

A catheter head for insertion into a body tissue is preferably provided which is divided into an insertion area and an adjoining connection area. The insertion area comprises several apertures in its surface. Moreover, the insertion area has a diameter that increases in the direction of the connection area. The apertures are provided in the insertion area in such a way that webs extending in the direction of the connection area are formed between the apertures along the surface of the catheter head. The webs begin in an area of the insertion area with a small diameter and end in an area with a greater diameter.

This design of the catheter head permits gentle and largely atraumatic insertion of the catheter head into and through the body tissue, particularly a cerebral tissue. During insertion of the catheter head, the tissue is initially spread apart only by the frontmost area and by the surface of the webs. The tissue is not yet stressed in the area of the apertures between the webs. After this initial spreading, the catheter head is inserted farther and the tissue areas at the apertures are also widened by the circumferential surface of the connection area. In this procedure, only minimal direct pressure is applied to the tissue.

The connection area of the catheter head is preferably circular or oval and has a uniform diameter. The diameter is preferably at most 3 mm. The insertion area is closed at the frontmost area of the tip and, for example, is parabola-shaped, round, or in the form of a truncated cone. The apertures can extend in an elongate shape from the insertion area to the connection area. Because of the increasing diameter of the insertion area, the webs between the apertures are curved in the longitudinal direction of the catheter. In a plan view of the tip, this results in a cross-shaped or star-shaped arrangement of the webs, depending on the number of apertures.

The apertures preferably have openings or are preferably formed completely by openings. The edges or boundaries of the apertures or openings are rounded or bevelled so as to provide a smooth transition of the adjoining surfaces. Inside it, the catheter head can have a channel which, together with a channel of the catheter, can form a drainage line in the proximal direction. The channel is connected to the openings that form the apertures. It is thus possible to use the openings as drainage openings for removal or delivery of fluid from or to the surrounding tissue. In order to form the drainage channel, a fastening piece suitable for fastening to a connecting pipe, preferably to a flexible tube part, of the catheter can adjoin the connection area in the longitudinal direction. The diameter of the connecting pipe or of the tube part on the fastening piece corresponds substantially to the diameter of the connection area, so as to give a smooth transition between these structural parts.

According to the invention, the recess consists of at least two windows. In a preferred embodiment these windows are located in an upper part of the stiff element, such as the lid.

The light emitter and light receiver can also be arranged in a stiff element, such as the tip element. The stiff element can also be the combination of the stiff element (such as the middle piece) and the tip element being combined with each other without having a flexible tube element in between. The structure and layout of the catheter device as described herein however remains the same. This embodiment is therefore claimed as a separate invention as well.

In another embodiment, only one of the light emitter and the light receiver is arranged in the stiff element, for example the middle piece, and the other one, preferably the light receiver, is arranged in the tip element. The first flexible tube part is preferably present between the tip element and stiff element or middle piece. This enables to variably change the distance between the light emitter and the light receiver. The light receiver is preferably a photodetector. The structure and layout of the catheter device as described herein however remains the same. This embodiment is therefore claimed as a separate invention as well.

In a variant of the embodiments described above, more than one light receiver, preferably more than one photodetector, is present, wherein the light receivers are arranged at a distance to each other. They can all be arranged in the stiff element, for example the middle piece, or in the tip element or some of them are arranged in the stiff element, for example the middle piece, and some are arranged in the tip element.

In a preferred embodiment, a smooth transition of the structural parts of the device is enabled by using a second tube consisting of an inner and an outer tube, wherein the stiff element is attached at least to the outer tube, preferably only to the outer tube, the outer tube thereby protruding the inner tube.

This kind of double tube can be used for other catheters and other kind of devices as well. For example, the light emitter and light receiver are arranged in the tip element and no stiff or middle element is present. In still another example, the stiff element or middle piece and the tip element are combined with each other without having a flexible tube element in between. In both examples, the inventive tube as described above can be used as well.

In an example not part of the invention the catheter is covered with a protection sleeve. The sleeve covers the catheter along its length. The protection sleeve comprises a distal end which protrudes a distal end of the catheter. The protection sleeve is open at its distal end and extends at a distance to the catheter located within the sleeve, wherein the protection sleeve is not transparent at least for visible light. This facilitates zero setting of a sensor of the catheter, especially of a light sensitive pressure sensor. This kind of protection sleeve can be used with other catheters as well, such as catheters with no stiff element and/or no middle piece but with light sensitive sensors arranged in a tip element.

More variants of the invention and additional inventive embodiments are described in the dependent claims. Note is made that combinations of the features of the different dependent claims can be made as well and are part of this disclosure in order to obtain additional embodiments not described in the following in detail.

### BRIEF DESCRIPTION OF THE FIGURES

An embodiment according to the present invention is shown in the drawings. Features of the measurement device that are evident from the figures are to be regarded as belonging to the scope of the disclosure and are not to be interpreted in any way as limiting the invention. In the drawings:
- FIG. 1: shows an overall view of a measurement device according to WO 2010/015094;
- FIG. 2: shows a three-dimensional view of a catheter head according to WO 2010/015094;
- FIG. 3: shows a front view of the catheter head according to Figure 2;
- FIG. 4: shows a longitudinal section through the catheter head according to Figure 3; and
- FIG. 5: shows a three-dimensional view of a first embodiment of a middle piece of a measurement device according to WO 2010/015094;
- FIG. 6a: shows a longitudinal section through a measurement device according to Figure 1 along a first longitudinal plane;
- FIG. 6b: shows a longitudinal section through a measurement device according to Figure 1 along a second longitudinal plane perpendicular to the first plane;
- FIG. 7: shows a three-dimensional view of a second embodiment of a middle piece of a measurement device according to WO 2010/015094;
- FIG. 8: shows a longitudinal section through a measurement device from Figure 1 according to the second embodiment;
- FIG. 9: shows a perspective view of a middle piece according to WO 2010/015094;
- FIG. 10: shows a perspective view of a lid of the middle piece according to Figure 9;
- FIG. 11: shows a view of the distal end of a lower part according to Figure 9;
- FIG. 12: shows a view of the proximal end of the lower part of the middle piece according to Figure 9;
- FIG. 13: shows a perspective view of the lower part according to Figure 12;
- FIG. 14: shows a top view of the lower part according to Figure 12;
- FIG. 15: shows a longitudinal section through the lower part according to Figure 12 with optical conductor and circuit board;
- FIG. 16: shows a schematic view of the measurement device according to WO 2010/015094;
- FIG. 17: shows a three-dimensional view of a catheter head according to WO 2010/015094, in another embodiment;
- FIG. 18: shows a measurement device according to the invention;
- FIG. 19: shows an enlarged perspective view of a part of the embodiment according to figure 18;
- FIG. 20: shows the measurement device according to figure 19 in an exploded view;
- FIG. 21: shows a longitudinal section of a middle piece according to figure 19;
- FIG. 22: shows an enlarged longitudinal sectional view of a proximal end of the measurement device according to figure 18;
- FIG. 23: shows an enlarged longitudinal sectional view of the middle piece of the measurement device according to figure 18;
- FIG. 24: shows an longitudinal enlarged sectional view of a distal end of the measurement device according to figure 18;
- FIG. 25: shows a side view of a part of the measurement device according to figure 18;
- FIG. 26: shows a cross-sectional view along the plane A-A of figure 25;
- FIG. 27: shows a cross-sectional view along the plane B-B of figure 25;
- FIG. 28: shows a cross-sectional view along the plane C-C of figure 25;
- FIG. 29: shows a perspective view of a lower part of the middle piece according to figure 20;
- FIG. 30: shows a perspective view of an upper part of the middle piece according to figure 20,
- FIG. 31: shows a perspective view of the middle piece of figure 20;
- FIG. 32: shows a side view of the middle piece of figure 31;
- FIG. 33: shows an enlarged cross-section of a second tube part and the middle piece;
- FIG. 34: shows the measurement device of figure 18 with a protection sleeve;
- FIG. 35: shows a longitudinal sectional view of the device and the sleeve of figure 34;
- FIG. 36: shows an enlarged view of part X of figure 35;
- FIG. 37: shows an enlarged view of part Y of figure 35 and
- FIG. 38: shows a cross-sectional view along B-B of figure 35.

### DETAILED DESCRIPTION

In the following description, the end with the catheter tip is designated as the distal end of the catheter probe, and the opposite end is designated as the proximal end.

In the following, the embodiments according to WO 2010/015094 are described and in addition, the invention is described based on these embodiments.

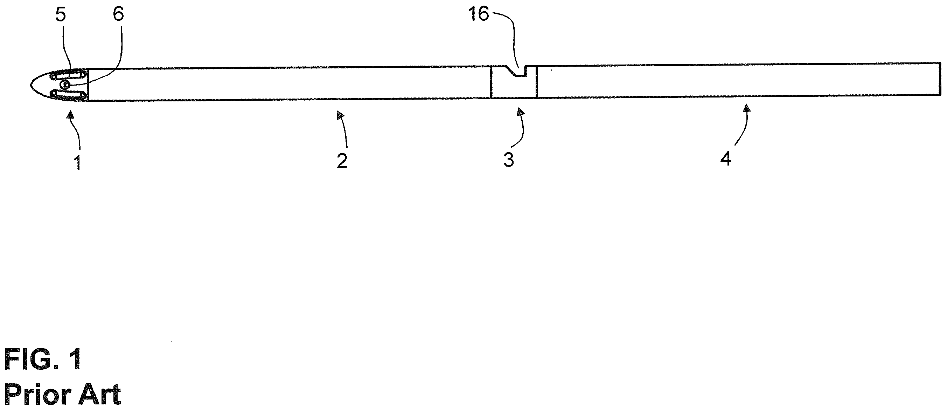

Figure 1 shows a measurement device according to WO 2010/015094 which has a tip element 1 as catheter head, a first connection tube 2, a middle piece 3, and a second connection tube 4. The first and second connection tubes 2, 4 are each preferably made from a flexible tube part. The text below therefore refers to a tube part, although this is also understood to mean other types of connection tubes 2, 4 that permit insertion of the catheter into the body tissue.

The tip element 1 is provided at the distal end of the measurement device. At the proximal end, the measurement device has a plug connector (not shown) or is guided directly into a processing unit for conversion and evaluation of the measurement signals. The tip element has several elongate openings 5, which form apertures in the surface of the catheter head. The openings 5 are provided for drainage of fluid in the surrounding tissue. Moreover, a round opening 6, in which a pressure sensor can be arranged, is provided between two elongate openings 5. However, the pressure sensor can also be arranged adjacent to, but not between, these irrigation openings 5. Electronic or optomechanical means, for example a silicon micromembrane, can be used as pressure sensors. The middle piece 3 has an indentation or aperture 16 into which a light conductor opens and through which light, delivered through the light conductor, can be emitted into the surrounding medium. The first tube part 2 can be varied in length depending on the intended nature of use of the measurement device.

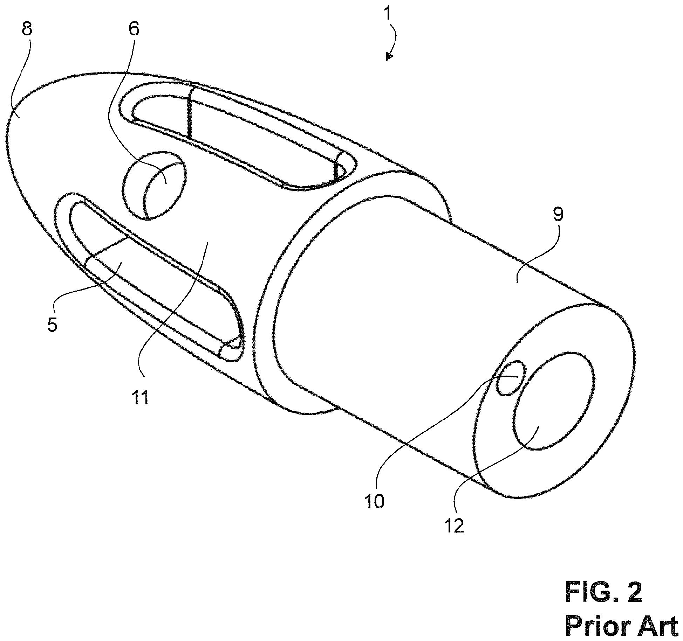

Figure 2 shows a three-dimensional view of the catheter head in the form of the tip element 1. The tip element can be made of plastic or of metal. The tip element is divided into a distal insertion area and an adjoining connection area. In the insertion area, it has an oval, parabola-shaped or round tip 8. The insertion area has a diameter increasing from the tip 8 in the direction of the connection area. The tip 8 is closed at its centre. It is followed shortly thereafter by one or more elongate apertures or openings 5, which are arranged alongside one another in the circumferential direction. The embodiment shown in Figure 2 has four openings, of which two can be seen in Figure 2. Between the elongate openings 5, webs 11 are formed in the circumferential area and extend from the connection area into the tapering area of the tip 8 and are thus slightly curved. The edge areas of the elongate openings 5 are rounded or trimmed, so as to give a gentle transition from the circumferential surface of the catheter head to the edge areas of the openings 5. The slight curvature of the webs means that, in the tip area, they run towards the centre or radially towards one another. The space between two adjacent elongate openings 5, which space forms the webs 11, is approximately as wide as the width of one elongate opening. Because of the slight curvature of the webs, this circumferential area tapers in the direction of the tip.

At the proximal end, the tip element 1 has a fastening piece 9, which is adjoined by the first tube part 2. The fastening piece 9 is part of the connection area or adjoins the latter. The fastening piece 9 is designed like a sleeve. A tubular structure can be placed with a form fit over the circumference of the fastening piece 9. In the circumferential wall of the fastening piece 9, a channel 10 extends in the longitudinal direction all the way from the distal end into the insertion area. The channel 10 serves for the passage of pressure measurement elements. For example, electrical or optical leads can be routed through the channel 10 as far as the opening 6, in which a pressure sensor can be arranged.

The elongate openings 5 serve for the drainage of the surrounding tissue. The drainage fluid is conveyed through an axially extending channel 12, which extends into the insertion area of the catheter head. It is an advantage that the pressure sensor can be arranged between the elongate openings 5 and therefore carries out a pressure measurement at the same level in the surrounding tissue where a fluid can be discharged via the drainage opening.

If the tip and the middle piece are made of plastic, the tip preferably has an X-ray marking, so as to allow the positioning of the tip to be checked.

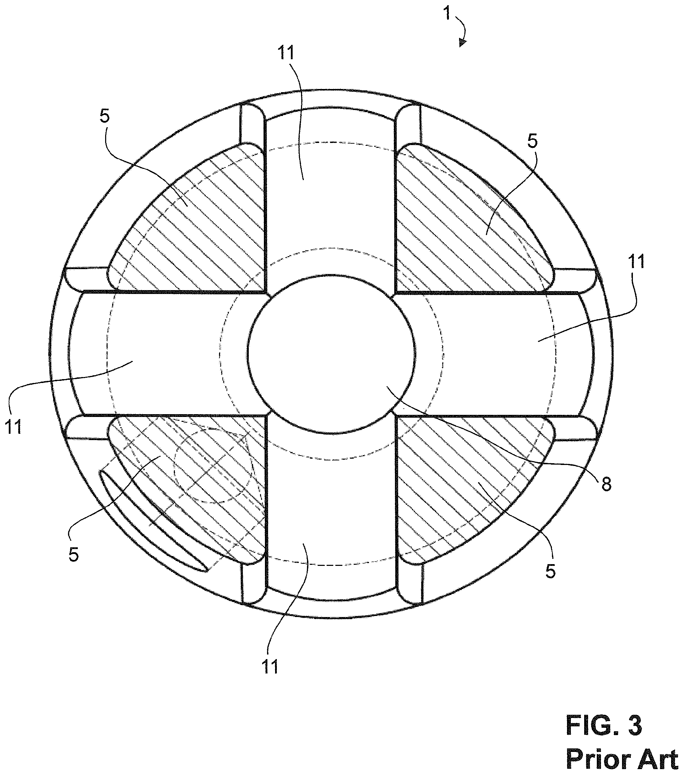

Figure 3 shows a schematic front view of a tip element 1. At the centre, the tip 8 can be seen as the frontmost curve of the catheter head. Arranged around this centre are the four apertures in the form of elongate openings 5 which, in plan view, form a free space between each other. The circumferential areas between the elongate openings 5 form the webs 11, which extend from the centre of the tip 8 to the outer circumference of the connection area and form a kind of guide structure for the tip element 1 upon insertion into a cerebral tissue. The webs 11 appear in a cross-shaped arrangement in the plan view in Figure 3. When the catheter head is inserted into the tissue, the tip 8, whose diameter is smaller than the diameter of the overall tip element, and the surface of the webs 11 are pressed directly onto the tissue. At first, no pressure is applied to the tissue in the area of the apertures, that is to say in the area of the elongate openings 5. The tissue is gently braced and spread apart by the webs 11, such that the catheter head can penetrate into the tissue and, in doing so, causes minimal trauma. It is only after the tissue in the insertion area of the tip element 1 has been initially widened by the webs 11 that the tissue is spread open completely across the entire diameter of the connection area. By means of this design of the catheter head of the measurement device, it is possible to insert the measurement device into interior areas of the brain virtually without injury.

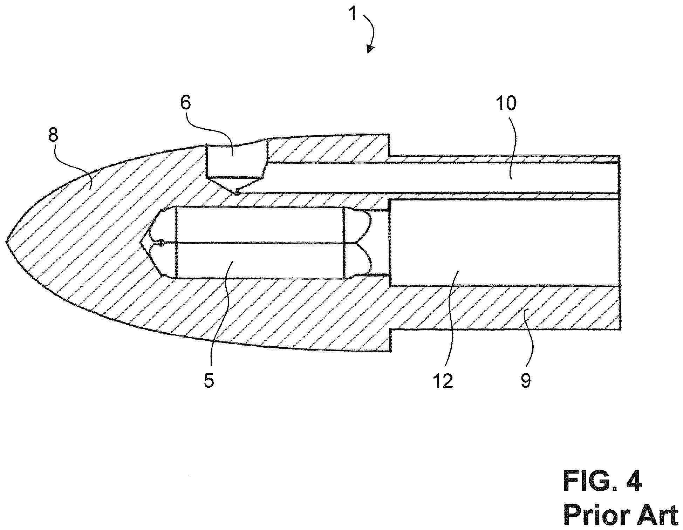

Figure 4 shows a cross section along the longitudinal axis of a tip element 1. It can be clearly seen that the tip element 1 in the insertion area and the tip 8 has a smaller diameter, which increases in the proximal direction as far as the connection area, which is adjoined by the fastening piece 9. The channel 10 extends in a straight line in the longitudinal direction as far as the opening 6, which is provided for a pressure sensor. In the interior, the four elongate openings 5 are adjoined by a guide channel 12, which is provided for removal of drainage fluid. In this view, the frontmost area of the tip 8 is shown as being pointed, which ensures that the widening of the tissue is optimized while no damage can be caused by scratching or cutting. The tip 8, or the cross section through the webs 11 of the catheter head, is substantially parabola-shaped.

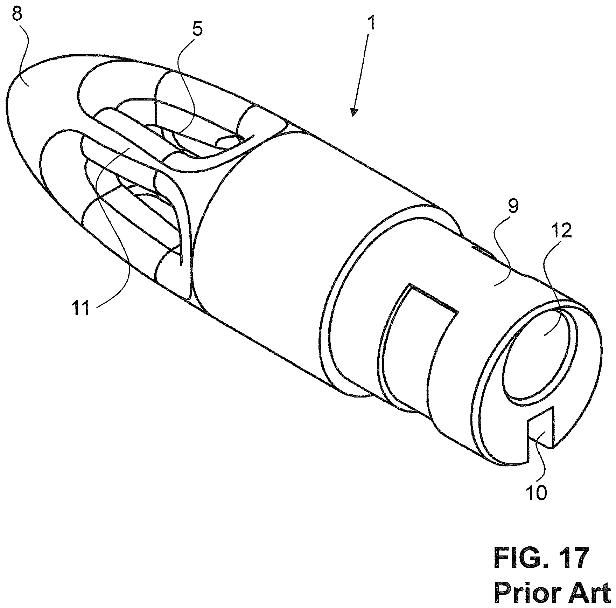

Figure 17 shows an embodiment of a tip element 1 in an alternative to Figure 2. Identical parts are provided with identical reference signs. The tip 8 is once again rounded. The openings 5 lie closer to one another than in the embodiment according to Figure 2, and they are separated from one another only by short webs. The webs preferably have a width that is a multiple smaller than the width of the individual openings. The drainage channel 12 extends axially offset in relation to the longitudinal centre axis of the tip part 1. The pressure sensor channel 10, or the channel for other electrical lines, is in this case open. The pressure sensor is preferably arranged on that side of the openings 5 lying opposite the tip 8 or is located in the middle piece.

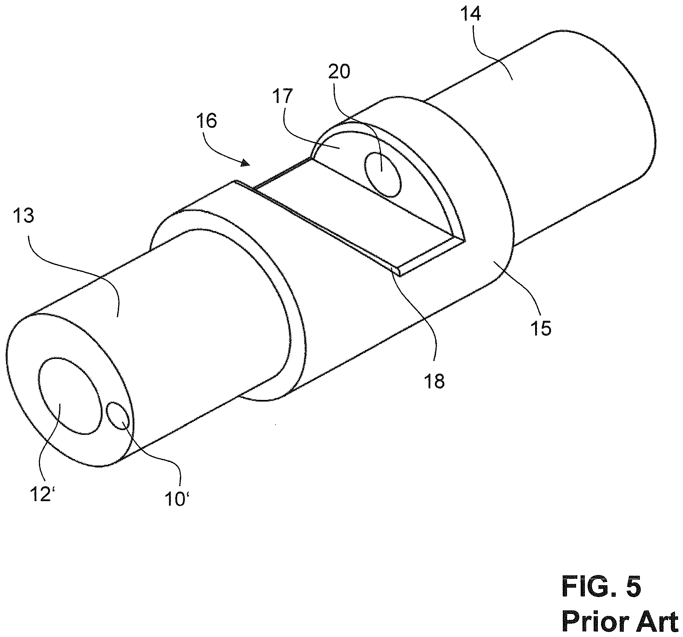

Figure 5 shows a middle piece 3 according to WO 2010/015094. The middle piece 3 has a distal fastening piece 13 and a proximal fastening piece 14. The fastening pieces 13 and 14 are similar to the fastening piece 9. They are sleeve-shaped and have a central passage for a drainage channel 12' for drainage of fluid and, in their circumferential wall, they have a continuous channel 10 for leads serving the pressure sensor in the tip element 1. The guide channel or drainage channel 12' in the middle piece 3 is analogous to the channel 12 in the tip element 1 and forms a continuation thereof.

On the middle piece 3, a middle area 15 is formed between the proximal fastening piece 13 and the proximal fastening piece 14. In the embodiment according to Figure 5, the middle area 15 has a wedge-shaped indentation 16 which, instead of extending all the way to the drainage channel 12', leaves the latter closed. At its proximal end, the indentation 16 extends with a light exit surface 17, from which an optical conductor emerges, perpendicular to the longitudinal axis. At its distal end, the indentation 16 has a reflection surface 18, which preferably extends at a 45° angle to the longitudinal axis of the middle piece 3 and to the surface 17. A channel 20, which ends in the light exit surface 17 of the indentation 16, extends through the circumference of the distal fastening piece 14 and the proximal end of the middle piece 3, in the longitudinal direction of said middle piece 3. The opening of the channel 20 in the indentation 16 thus lies opposite the oblique surface 18. The channel 20 is provided for guiding a light conductor. The light conductor can also extend past the light exit surface and protrude into the indentation 16. The light from the light conductor strikes the opposite reflection surface 18. A mirror or other type of reflector, arranged on the reflection surface 18, reflects the light from the light conductor into the surrounding tissue around the middle piece 3. The reflector can be mounted as a separate element on the reflection surface 18, or the reflection surface 18 itself can be configured as a reflector surface. For example, a gold coating can be provided as reflection surface. It is also possible for the surface 18 or the reflector to have a slightly curved shape, such that the incident beam of light is widened slightly. The indentation 16 is filled with epoxy resin, such that the surface of the middle area 15 is cylindrical.

An individual optical fibre is preferably suitable as light conductor. There are preferably not more than five optical fibres present. Two or three fibres can likewise be used.

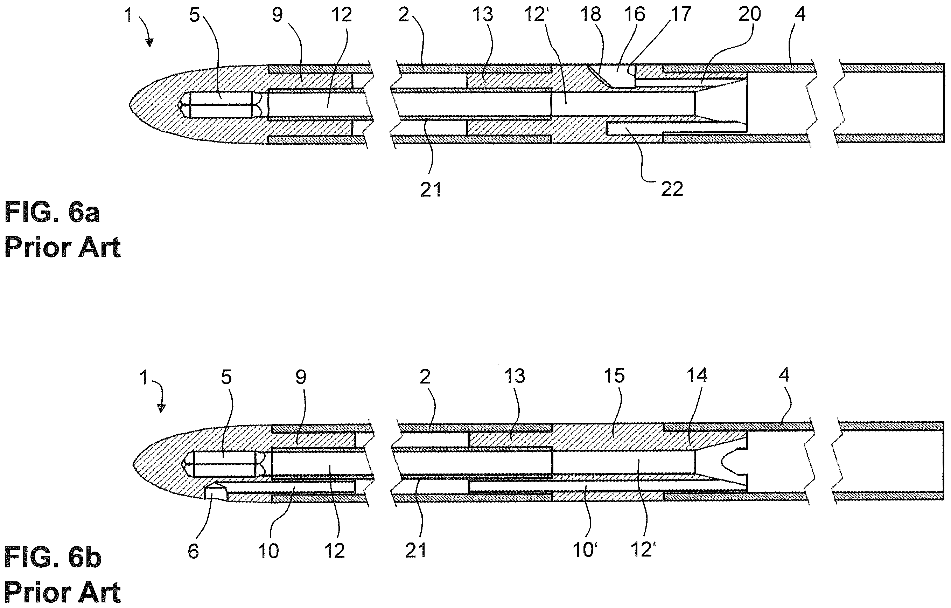

Figures 6a and 6b each show longitudinal sections through a first embodiment of a catheter probe according to WO 2010/015094, these longitudinal sections being offset by 90° to each other. Figure 6a shows, from left to right, a tip element 1, a first tube part 2, a middle piece 3 and a second tube part 4 according to the view from Figure 1. The first tube part 2 has one end pushed over the fastening piece 9 of the tip element 1 and has the other end pushed over the fastening piece 13 of the middle piece 3. In the interior of the first tube part, between the tip element 1 and the middle piece 3, there is another flexible tube 21 that interconnects the guide channels 12 and 12' of the tip element and of the middle piece. The channels 12 and 12' form, together with the tube 21, a drainage channel for fluid that is intended to be removed from the tissue surrounding the catheter tip, by way of the openings 5. The length of the tube parts 4 and 21 can be varied, such that the position of the middle piece 3 in the tissue can also be adjusted, i.e. tube parts of different lengths can be fitted between the tip element and the middle piece.

The wedge-shaped indentation 16 with the light exit surface 17 and the reflection surface 18 can be seen on the middle piece 3. The channel 20 opens into the light exit surface 17. The light exit surface 17 extends substantially perpendicular to the longitudinal axis of the catheter, although it could also be arranged at an angle thereto. An optical conductor, guided through the channel 20, is guided onwards through the second tube part 4 to a light source (not shown).

A temperature measurement channel 22 is also provided in the circumferential wall of the middle piece 3, on the proximal side, said temperature measurement channel 22 beginning on the side of the second tube part 4 and ending roughly at the centre of the middle piece 3. The temperature measurement channel 22 is provided for a temperature sensor that measures the temperature of the surrounding tissue. The temperature sensor is arranged at this point near the outer periphery of the catheter and is able to measure the temperature in the tissue without impediment or distortion. Leads (not shown) run from the temperature sensor through the second tube part 4 to the processing unit and transmit a temperature signal to the processing unit.

Figure 6b shows, within the tip element 1, the pressure sensor channel 10 which extends from the proximal end of the tip element 1 as far as the opening 6. In the opening 6, there is a pressure sensor whose leads run through the channel 10 and the space between the first tube part 2 and the inner tube 21 as far as a channel 10' in the middle piece 3. At the proximal end of the middle piece, the leads emerge from the channel 10' and are routed further through the second tube part 4 as far as the processing unit, to which they transmit a signal corresponding to the pressure in the medium surrounding the tip element 1 or in the guide channel 12. The processing unit is able to control the drainage of fluid through the drainage channel depending on the pressure signal from the pressure sensor. Manual drainage can also be performed.

The channel 12' on the middle piece 3 is widened conically at its distal end. This funnel-shaped opening facilitates insertion of a guide wire, which is used to guide the catheter probe during insertion into the body tissue.

The optical light conductor and the leads for the pressure sensor and temperature sensor can be routed freely within the interior of the second tube part 4. However, an inner tube can also be provided in this tube part, such that the leads are routed through the space between the outer tube and inner tube. The second tube part 4 can be adjoined by a plug in which the leads end. The plug can be fitted directly to the processing unit or to another plug, which in turn leads to the processing unit.

When examining a body tissue, for example the cerebral tissue in the interior of the brain, an opening is made in the cranium. A holder for the catheter is provided over the opening and holds the catheter as far as possible perpendicular to the surface of the head during insertion and also in the inserted state. Thus, the holder on the one hand covers the opening in the head and on the other hand ensures that the catheter is not accidentally deflected from its intended path during its insertion and during measurements. In this way, it is possible to avoid contamination of the area being examined and to avoid unnecessary damage to the surrounding tissue.

The catheter probe is inserted carefully through the tissue as far as a desired measurement position, for example until it rests on the floor of a ventricle. The design of the catheter head means that in doing so there is only a minimal impact on the cerebral tissue, such that the tissue is not unnecessarily damaged and the measurement is not distorted.

To measure the flow of blood through the cerebral tissue, light in the near infrared range is conveyed through the light conductor in the channel 20 to the reflection surface 18. The light source can, for example, also be provided on the processing unit itself. The beam of light directed onto the surface 18 is reflected there and sent out into the cerebral tissue. There, it is absorbed and reflected among other things on the oxygenated and deoxygenated haemoglobin and, if appropriate, on the indocyanine green. Some of the reflected light falls back onto the reflection surface 18, as a result of which it is fed into the light conductor in the channel 20 by reflection on the surface 18. The channel guides the reflected light back to the processing unit, in which it is evaluated by, for example, an analog/digital converter. At the same time, the temperature in the measured tissue can be determined by the temperature sensor in the channel 22, and the pressure in the area surrounding the tip of the probe can be determined by the pressure sensor. If necessary, drainage can be performed immediately via the channel 12, 12'.

By means of the measurement probe being guided by the holder, the probe can be turned in its measurement position without thereby being deflected and damaging the surrounding tissue. By turning the probe, the measurement range in the tissue can be greatly widened. It is possible to cover a measurement field that extends through 360° around the middle piece 3. The tube parts between the tip and the middle piece can be flexible or rigid.

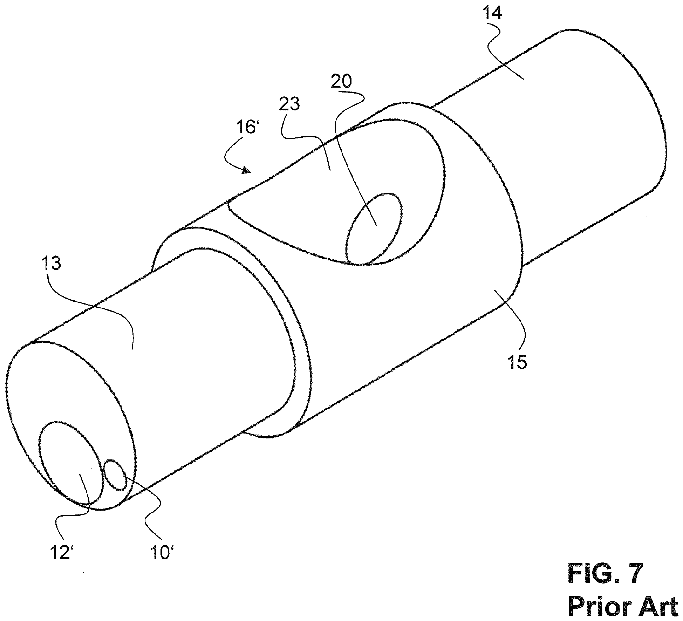

Figure 7 shows a second embodiment of a middle piece according to WO 2010/015094. In this design of the middle piece, the drainage channel 12' is eccentric to the longitudinal axis. In this way, the middle piece can be constructed with a smaller diameter. Moreover, the recess is provided here in the form of an aperture 16' which is sunk in a curved or rounded shape into the middle piece. An inner surface 23 thus formed comprises a light exit surface area at the proximal area, and, lying opposite this at the distal area, it has a reflection surface area. In terms of its function, the light exit surface area corresponds to the light exit surface 17 from the embodiment according to Figures 1 to 6, and the reflection surface area accordingly corresponds in terms of its function to the reflection surface 18. The light-conducting channel 20 opens out from the light exit surface area. Light emitted from an optical conductor in the light-conducting channel 20 strikes the opposite reflection surface area, from which it is reflected into the surrounding tissue. The curvature of the aperture 16' is designed such that light reflected from the tissue is focussed into the optical conductor.

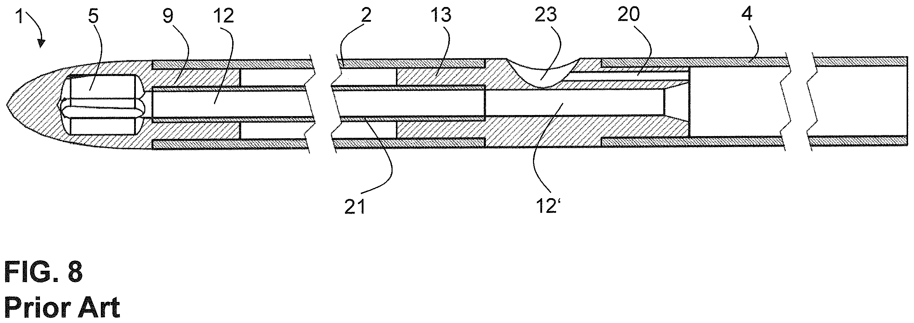

Figure 8 shows a longitudinal section through a measurement device with a middle piece according to Figure 7, but the drainage channel 12' is arranged in a central position here. The aperture 16' reaches almost as far as the drainage channel 12' but leaves the latter closed. The curvature or roundness of the aperture 16' is such that light from the channel 20 is oriented directly onto the focal point. The other elements of the measurement device in this embodiment correspond to those from Figures 6a and 6b.

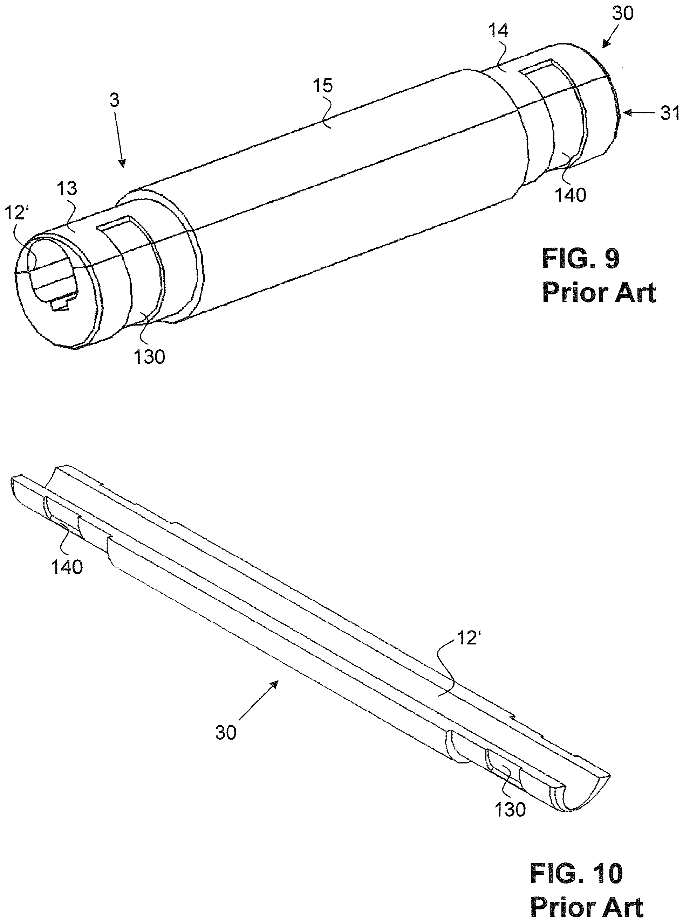

Figures 9 to 14 show the middle piece 3 according to WO 2010/015094in a preferred embodiment. Identical parts are provided with the same reference signs as in the above examples. The middle piece 3 is made of plastic and is preferably produced by injection moulding. It is composed of at least two parts, namely a lower part 31 and a lid 30. Both are configured as half tubes, as can be seen in Figure 10 for the lid 30. Together they form a tubing with a middle area 15, a distal fastening piece 13 and a proximal fastening piece 14. Grooves 130, 140 are provided extending radially about the circumference of the two fastening pieces 13, 14 in order to permit a connection to adjoining tubes. These grooves serve as adhesive grooves for a form fit and force fit. An equivalent groove can be seen in Figure 17.

Instead of flexible tubes, rigid connector parts can also be present on both sides or on one side.

In contrast to the above examples, the recess in which the mirror, the fibre end and any measuring means are arranged is now covered by the lid 30. The lid 30 can be connected to the lower part with a form fit and/or a force fit and/or a materially cohesive fit.

Lid 30 and lower part 31 are preferably made from the same material. At least the lid 30, however, must be made from a material that is transparent for the optical wavelength used. They are preferably made of polyamide or polycarbonate.

As can be seen from Figure 9, the lower part 31 and the lid 30 form a common inner drainage channel 12' with a cable channel 120. The drainage line already described above and any measurement lines extend through this drainage channel 12' as far as the distal head part of the catheter.

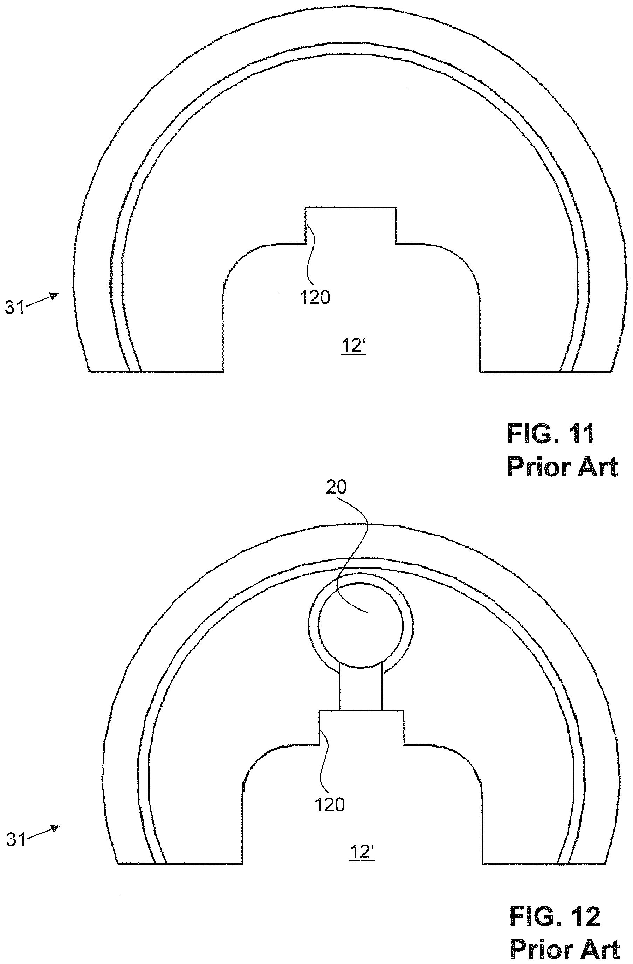

Figure 11 shows the distal end of the middle piece 3 with the drainage channel 12'. Figure 12 shows the inner proximal end of the middle piece 3 not visible in Figure 9. A light conductor channel 20 extends parallel to the drainage channel 12' but spaced apart therefrom. Here too, it is preferable to use exactly one optical fibre as light conductor. However, there can also be up to five optical fibres, in particular two or three.

Under the aperture 16, the drainage channel 12' extends along the entire length of the middle piece 3.

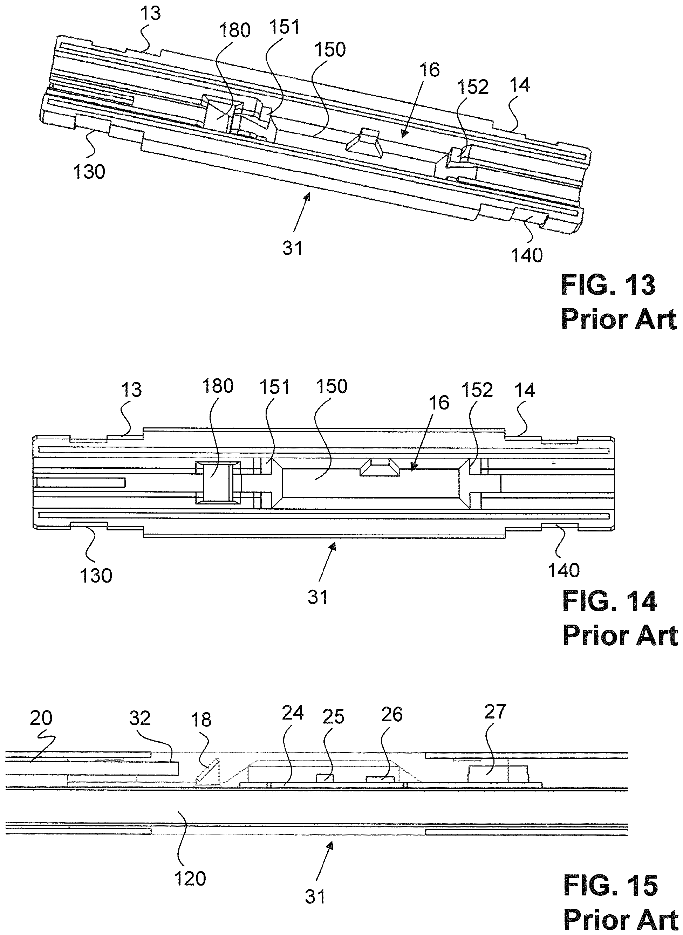

Figures 13 to 15 show the lower part 31 in detail. It has the aperture 16. A receiving area 150 for the electronics is present in this aperture 16. Proximal and distal receiving surfaces 151, 152 are formed integrally on both end areas of the receiving area 150. A circuit board 24 can be secured, for example adhesively affixed or screwed, onto these plane receiving surfaces 151, 152.

A photodetector 25 can be secured on the circuit board 24. It is also possible for a temperature sensor 26 and/or a pressure sensor 27 to be present. However, these sensors can also be arranged at another location. As has already been mentioned above with reference to the other examples, the pressure sensor is preferably arranged in the tip element 1.

An inclined surface 180 is present outside this receiving area 150. This inclined surface 180 can itself be designed as a reflection surface. However, it preferably serves as a holder for a mirror 18. The mirror 18 in this example is a plane-parallel mirror, such that the inclined surface 180 is also designed as a plane surface. The inclined surface 180 is preferably oriented at a 45° angle to the longitudinal direction of the middle piece 3.

The light conductor channel 20 extends in the lower part 31 and ends adjacent to the inclined surface 180. The light conductor 32 is shown in Figure 15. It will be seen that it ends before the mirror 18, such that light emitted from the light conductor 32 is deflected from the mirror 18 and conveyed radially outward.

In this example, light reflected in the tissue is no longer fed back via the light conductor and evaluated. It is instead detected by the photodetector, and an electrical signal is transferred to an external evaluation unit via a signal line. The cables necessary for this purpose run inside the cable channel 120.

This middle piece 3 can be used with one of the tip elements 1 described above. However, it can also be used with other tip elements 1.

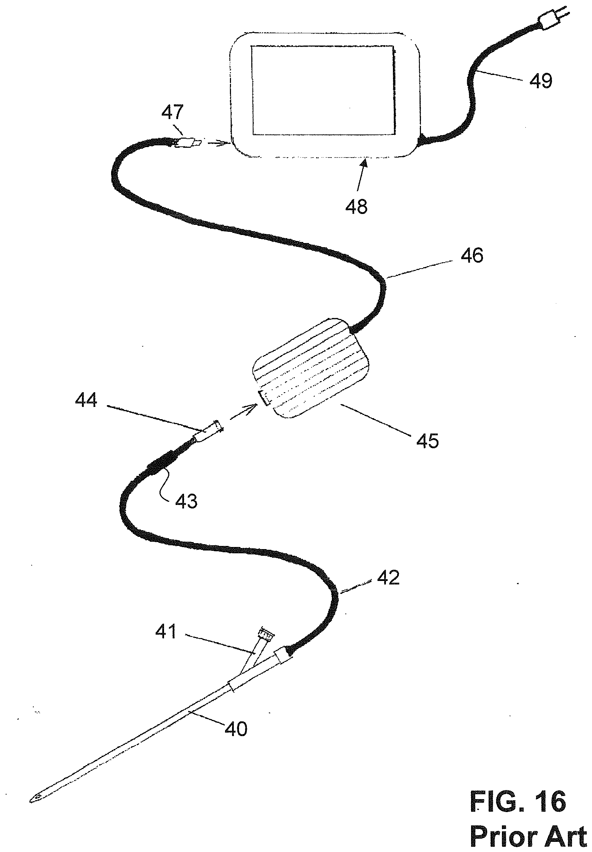

Figure 16 shows the overall system. The catheter or the probe 40 is provided at the end of the drainage channel with an y-connector with a Luer lock. A first line 42, which comprises electrical conductors and also at least one optical conductor, is connected with a distal end to the probe 40. In the area of the proximal end, it has a calibration unit 43, and it is provided at this end with a first plug 44. This plug 44 permits a connection to a portable electronics unit 45. This electronics unit 45 preferably comprises a potential insulator, an A/D converter, a pre-amplifier, and a light source for coupling light into the optical conductor that leads to the middle piece 3 of the catheter.

The portable electronics unit 45 is to an evaluation unit 48 via a second, purely electrical line 46, which in turn is provided with a second plug 47. A third line 49 represents the mains supply cable. However, the evaluation unit 48 can of course also be battery-operated.

The advantage of this overall system is that the electronics unit 45 is relatively close to the patient. The sensitive optical lines can therefore be made relatively short.

In the examples described above, an optical glass fibre is preferably used in the near infrared range. Its diameter is preferably approximately 0.6 mm inclusive of its sheath and approximately 0.2 mm without its sheath. The length of the middle piece 3 is preferably 20 to 30 mm, and its diameter is preferably 3 mm. The mirror surface preferably measures 1x1 mm. The drainage channel preferably has a diameter of 0.9 to 1.5 mm, preferably 1.0 or 1.2 mm. The width of the circuit board is preferably 1.4 mm. A 9-pole or 10-pole cable, which extends as far as the plug 44, is normally used inside the middle piece 3.

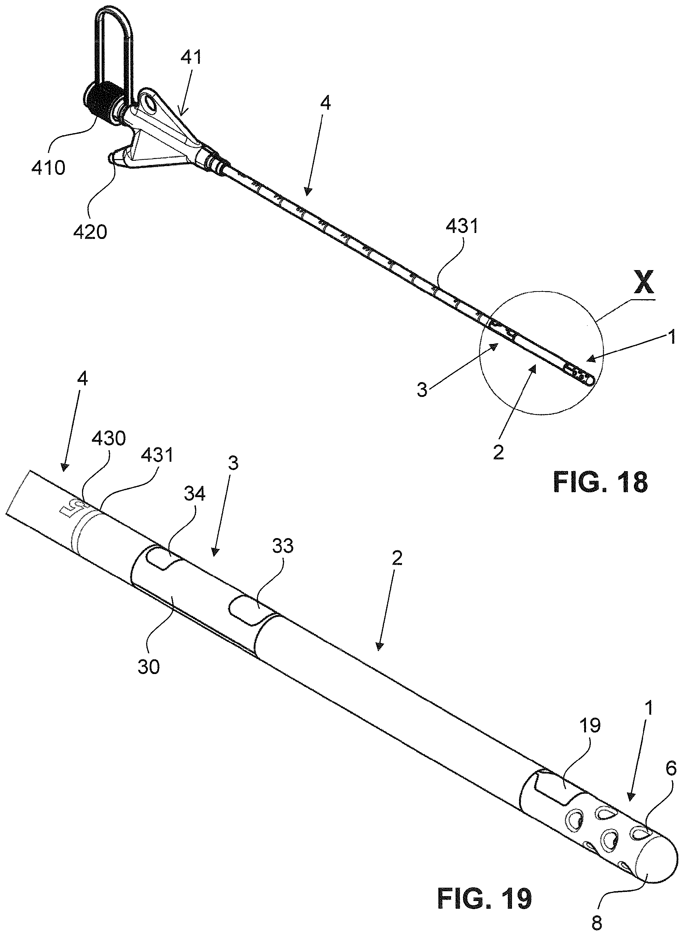

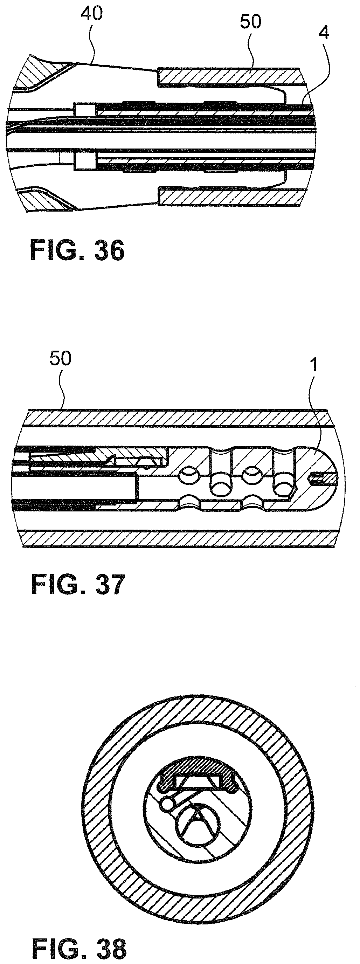

Figures 18 to 38 show an inventive measurement device. Same elements have the same reference numbers as the embodiments according to WO 2010/015094.

The measurement device comprises, as shown in figure 18, a tip 1 as described above, the first tube part 2, the middle piece 3 and the second tube part 4 ending in the y-connector 41. The y-connector 41 comprises two or more ports 420, wherein at least one of them is closed by a cap 410. The middle piece can be a stiff element which is not a middle piece but which is arranged at the end of the catheter instead. However, in the following description, the expression "middle piece" will be used when "stiff element" in a more general way of placement is meant as well.

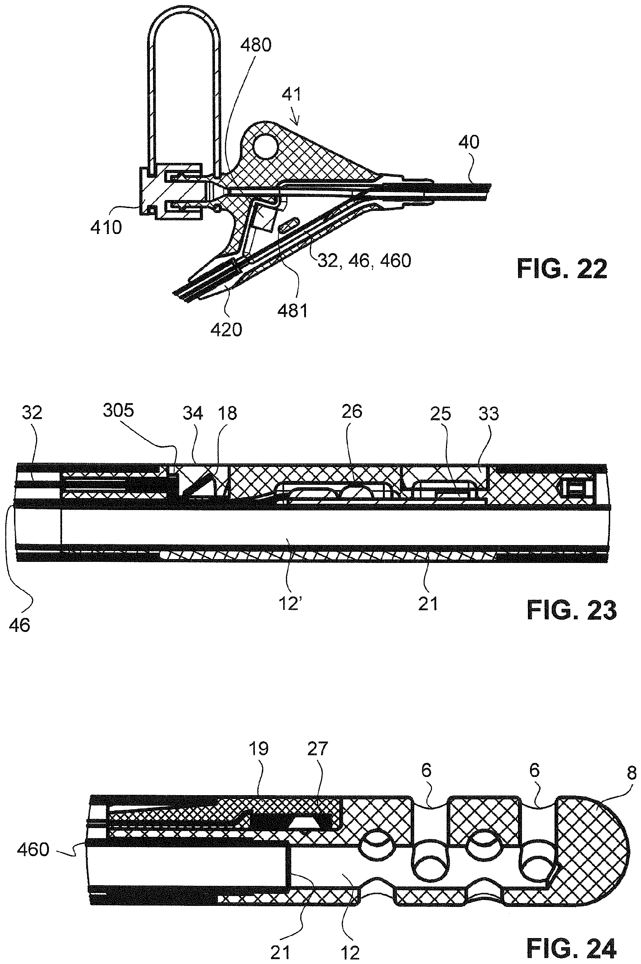

As can be seen in figure 22, the light conductor 32, the purely electronic line 46 and a pressure sensor line 460 extend through the open port 420, wherein the closed other port is used for introducing guide wires and/or for fluid transfer.

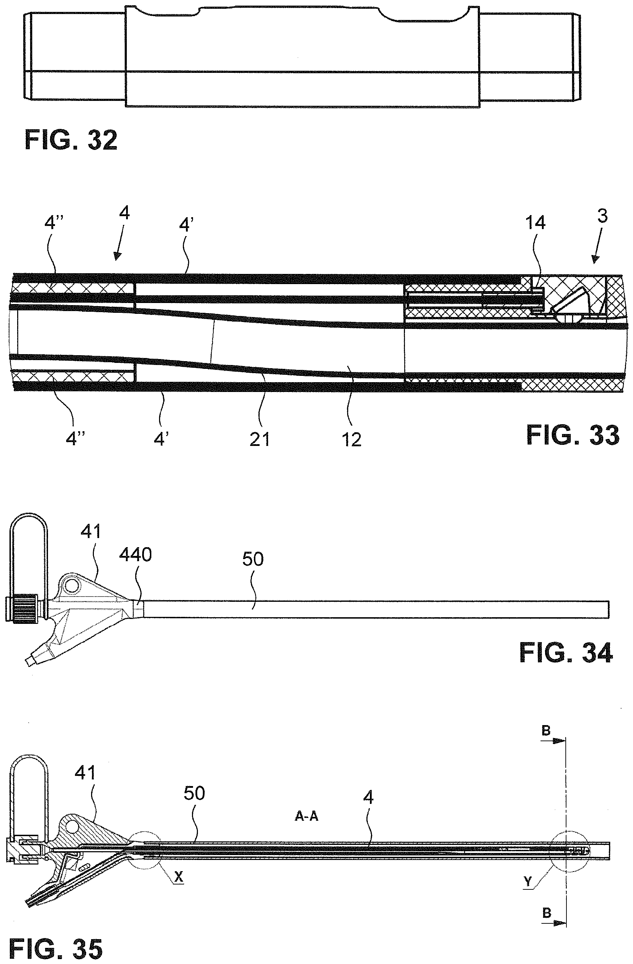

As also shown in figure 22, the y-connector 41 may comprise a data chip 480, such as an EEPROM, which identifies the device. For example, it can comprise information about the sensors, calibration data, date about the first use of the device and manufacturing data. Reference number 481 refers to a mounting aid for the light conductor 32, the second line 46 and the pressure sensor line 46''. It is for example a nose protruding into the inner space of the y-connector 41. The second tube part 4 preferably consists of two tubes, an inner second tube 4' and an outer second tube 4''. This can be seen in figure 33. The inner second tube 4' has a slightly smaller diameter than the outer second tube 4". They are preferably bonded to each other along at least a part of their length.

The distal end of the outer second tube 4" protrudes the distal end of the inner second tube 4' so that only the outer second tube 4" extends over the proximal fastening piece 14 of the middle piece 3 and is connected to it. The inner second tube 4' ends at a distance to the proximal fastening piece 14. This reduces the outer diameter of the device wherein the second tube part 4 has nevertheless a sufficient thickness to ensure stability of the tube when introduced into the patient. At the proximal end of the second tube part 4 the two ends of the inner and outer second tube 4', 4" preferably end in a common plane.

In other embodiments, they end in a step as well.

The first tube part 2 may be likewise made of an inner and an outer tube, the outer tube protruding the inner tube at least at the proximal end, preferably also at the distal end. In this embodiment however, the first tube part 2 is shaped like the well-known catheter tubes.

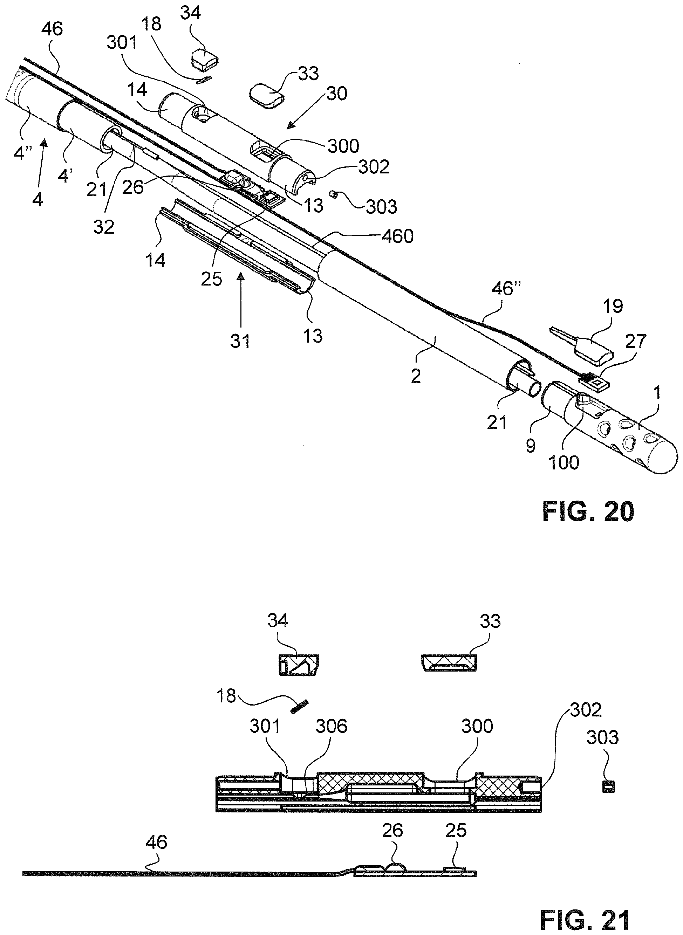

Note is taken that figure 20 shows the inner second tube 4' in a protruding state. This is for better view only and should not be in contradiction to the above mentioned description. In addition, the electronic lines and the fiber optic, i.e. the light conductor, extend outside of the first and second tube parts 2, 4, which is for better view as well. In the assembled device, they extend inside these tube parts 2, 4.

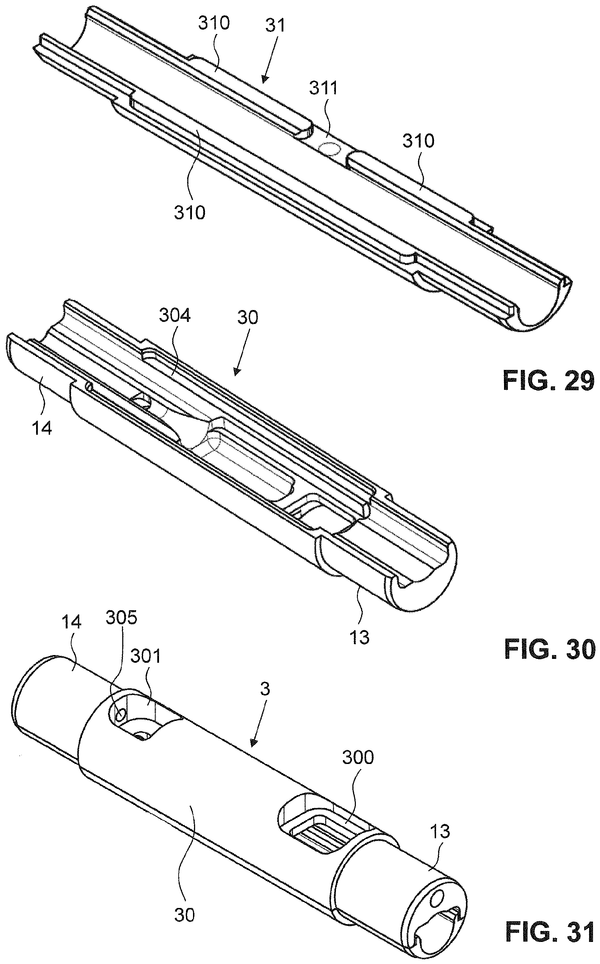

The middle piece 3 of this embodiment comprises a lid or an upper part 30 and a lower part 31 as can be best seen in figures 29 to 32. The two parts 30, 31 are preferably formed as half tubes which can be fixed to each other so that they form together a tube. As shown in figure 30, the upper part 30 comprises a notch 304 on both sides of this diameter. The lower part 31 as shown in figure 29 comprises a corresponding adjustment longitudinal nose 310 on both sides which engages this notch 304.

The middle piece 3 comprises the distal fastening piece 13 and the proximal fastening piece 14 for connection with the first tube part 2 and the second tube part 4 respectively. In the embodiment shown, an X-ray marker 303 is preferably placed in a hole 302 of the distal fastening piece 13, as shown in figure 20. In other embodiments, the X-ray marker is preferably arranged in the tip element 1 and more preferably at its distal end.

The inner side of the lower part 31 is preferably smooth and comprises not recesses or protrusions.

The upper part 30 of the middle piece 3 comprises two openings or windows 300, 301 which form two parts of a recess. The distal first window 300 is for receiving signals from the patient, the proximal second window 301 for emitting light into the patient. The first window 300 is closed with a sensor cover 33, the second window 301 is closed with a mirror holder 34 as shown in figures 19 to 21.

In other embodiments, the light source is directly placed in this second window 301 and no light conductor is present. In this case, the light source is preferably an LED or a laser chip, preferably a bare die.

The sensor cover 33 and the mirror holder 34 are preferably transparent for the at least one wavelength used in this device. Preferably they are made of polycarbonate. Preferably a wavelength of 808 nm and/or 905 nm is used. Other wavelength may be used as well.

The lower part 31 is preferably transparent as well so that it can be bonded to the upper part 30 more easily using UV light. Also the fixation of the elements arranged within the middle piece 3 can be established more easily when the lower part is transparent. Preferably the lower part is not only transparent for UV light but also for visible light so that elements arranged within the part are visible when mounted and fixed.

Preferably, the upper part 30 is made of none-transparent material. Preferably, it is made of PEEK (polyether ether ketone). A none-transparent upper part 30 has the advantage, that scattered or reflected light can only reach the photodetector 25 through the first window 300.

The upper part 30 comprises a channel with an outlet opening 305 directed to the second window 301. The light conductor 32 extends to this opening so that light can be guided along the longitudinal axis of the device to a mirror 18 arranged in the second window. The mirror 18 is fixed in the window. The window holder 34 holds the mirror 18 in a specific angle relative to the longitudinal axis of the device. A mounting hole 306 shown in figure 21 arranged at the bottom of the second window 301 helps to mount the mirror 18 at the predetermined place and angle.

A sensor plate is arranged below the first window 300. This sensor plate comprises at least the photodetector 25, but preferably also the temperature sensor 26 and/or other sensors. In a first variant, only one photodetector 25 is present. In other variants, more than one photodetector is present, especially when more than one wavelength is used.

As can be seen in figure 23, the at least one photodetector 25 is arranged in the first window 300 and covered by the sensor cover 33.

Using two windows instead of only one has the advantage, that the accuracy of the measurement is improved since no light can be forwarded within the middle piece 3from the light conductor to the photodetector. This arrangement optimizes the optical path.

The temperature sensor 26 is preferably arranged under the none-transparent region of the upper part 30 between the first and the second window 300, 301.

Preferably a diode is used as a temperature sensor. Since it is arranged in an area where no light can enter, the temperature measurement is not disturbed by emitted or reflected light.

As shown in figures 19, 20 and 24, the pressure sensor 27 is arranged in the distal tip element 1. Preferably it is a sensor chip and more preferably a bare die. In this embodiment, it is arranged at the proximal side of the drainage outlets of the tip element 1. In other embodiments it is arranged at the distal end of the tip element, i.e. in front of the drainage openings.

The tip element 1 comprises a cavity 100 being closed with a sealing element 19, such as a sealing compound made of silicone.

The pressure sensor 27 is arranged in this cavity 100, wherein the electronic pressure sensor line 46" extends from the sensor 27 along the device to the y-connector 41.

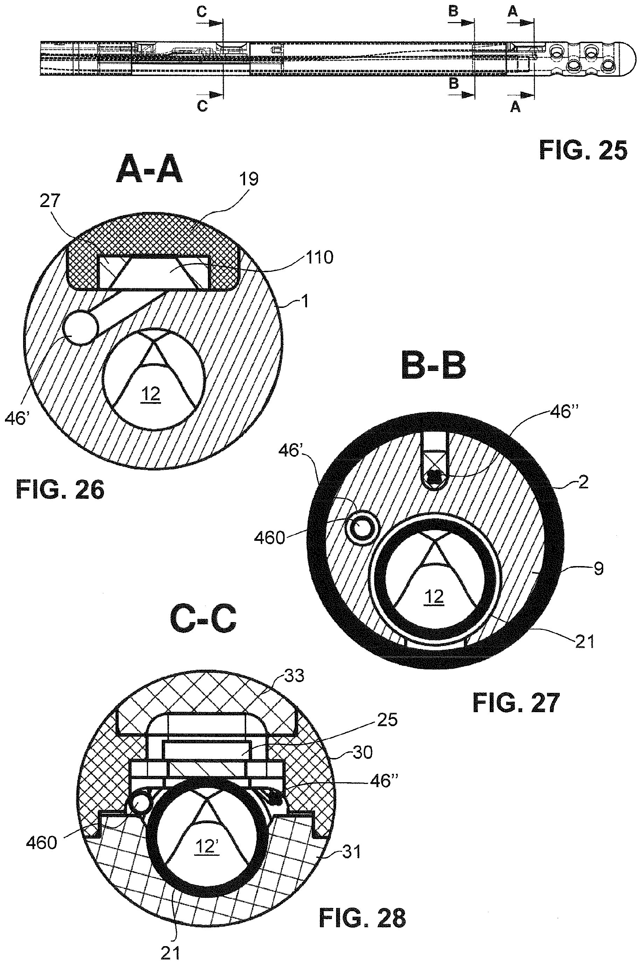

Figures 26 to 28 show cross-sections along the device, as shown in figure 25. In figure 26, the distal tip element 1 with the drainage channel 12 can be seen as well as the cavity 100 closed with the sealing element 19. As can be seen, a space 110 is present under the pressure sensor 27. The space 110 is connected to a pressure equalization channel 46' which extends to the outside of the device, so that the sensor 27 is exposed to ambient pressure. In other embodiments, the pressure sensor is an absolute pressure sensor or a fiber-optic sensor.

In figure 27, the distal end of the first tube part 2 is arranged over and bonded to the fastening piece 9 of the tip element 1, as can also be seen in figures 20 and 24.

In figure 27, the pressure equalization channel 46' and the pressure sensor line 46" can be seen. The pressure equalization channel 46' formed within the tip element 1 changes now into a pressure equalization tube 460 extending along the longitudinal axis of the device.

Figure 28 shows the first sensor cover 33 arranged over the photodetector 25, the pressure sensor line 46" and the pressure equalization tube 460 as well as the inside tube 21.

Figures 34 to 38 show an optional feature for the inventive measurement devices described above. The inventive measurement device is usually adjusted to zero shortly before use. It may also be calibrated as well. In the state of the art, such measurement devices are therefore immersed into water or another appropriate liquid. Since pressure sensors or also other sensors are light sensitive, the water reservoir has to be none-transparent for the relevant wavelengths. Usually it is black so that no visible light can reach the sensor during zero adjustment. It may also be non-transparent for other or only for other wavelength, depending on the sensibility of the pressure sensor to a specific wavelength.

It is an additional inventive idea to store the measurement device in a none-transparent protection sleeve 50 covering at least the sensor parts of the device. The protection sleeve is at least none-transparent for the relevant wavelengths, i.e. the wavelengths which the sensor is sensible of. Preferably, the protection sleeve is black.

Preferably the sleeve 50 is pushed over the distal end of the y-connector 41 and protrudes the distal end of the tip element 1. It is arranged at a distance to the tubes 2, 4, the middle piece 3 and the tip element 1, as can be seen in figures 35 to 38. This sleeve serves as protection when the device is transported.

For zero adjustment or calibration, the measurement device can be immersed together with the protection sleeve 50 in any kind of container, since no light can reach the sensor in any case. The sleeve 50 is only be removed from the measurement device shortly before the device is actually introduced into the patient. This simplifies the handling of the device and reduces mishandling.

Note is made that the embodiment according to figures 18 to 38 can be combined and varied following the teaching and disclosure of the previously described embodiments as well.

### List of reference numbers

- 1: tip element
- 100: cavity
- 110: space
- 2: first tube part

- 3: middle piece
- 30: upper part/lid
- 300: first window
- 301: second window
- 302: hole
- 303: marker
- 304: notch
- 305: outlet opening
- 306: mounting hole
- 31: lower part
- 310: adjustment nose
- 311: gap
- 32: light conductor
- 33: sensor cover
- 34: mirror holder
- 4: second tube part
- 4': inner second tube
- 4": outer second tube
- 5: elongate opening
- 6: round opening
- 8: tip
- 9: fastening piece
- 10, 10': pressure sensor channel
- 11: web
- 12, 12': drainage channel
- 120: cable channel
- 13: fastening piece, distal
- 130: distal adhesive groove
- 14: fastening piece, proximal
- 140: proximal adhesive groove
- 15: middle area
- 150: receiving area for electronics
- 151: proximal receiving surface
- 152: distal receiving surface
- 16, 16': recess, indentation, aperture
- 17: light exit surface
- 18: reflection surface/mirror
- 180: inclined surface
- 19: sealing element
- 20: light conductor channel
- 21: inside tube
- 22: temperature measurement channel
- 23: inner surface with light exit surface area and reflection surface area
- 24: circuit board
- 25: photodetector
- 26: temperature sensor
- 27: pressure sensor
- 40: probe
- 430: marking number
- 431: marking ring
- 440: stop collar
- 41: y-connector
- 410: cap
- 420: port
- 42: first line (optoelectronic)
- 43: calibration
- 44: first plug
- 45: portable electronics unit
- 46: second line (purely electronic)
- 46': pressure equalization channel
- 46": pressure sensor line
- 460: pressure equalization tube
- 47: second plug
- 48: evaluation unit
- 480: data chip (EEPROM)
- 481: mounting guide
- 49: third line
- 50: protection sleeve

## Claims

1. A cerebral tissue catheter for measuring a flow of blood through a cerebral tissue of a patient,
the catheter having a proximal end remaining outside of the patient and a distal end to be introduced into the patient,
the catheter comprising a first flexible connection tube (2, 4), a stiff element (3) comprising at least a first window (300) and a second window (301) and a guide channel (12, 12'), the guide channel (12, 12') extending from the proximal end to the distal end and the guide channel (12, 12') extending through the stiff element (3), the guide channel (12, 12') being capable of receiving a stiff wire, thereby enabling the stiff wire to be moved through the catheter,
wherein a light emitter for emitting light into the body tissue and at least one light receiver for receiving light reflected in the body tissue and back to the catheter are arranged in the stiff element (3), wherein the light emitted from the light emitter is emitted through the second window (301) into the body tissue and the light reflected in the body tissue is received by the at least one light receiver through the first window (300).

2. The catheter according to Claim 1, wherein the stiff element (3) comprises an upper part (30) and a lower part (31) being connected to each other, wherein the at least first and second window (300, 301) are arranged in the upper part (30), wherein the upper part (30) is not transparent for the wavelength used.

3. The catheter according to Claim 2, wherein the lower part (31) is transparent at least for a wavelength used during assembly of the lower and upper part (30, 31).

4. The catheter according to one of Claims 1 to 3, wherein the stiff element (3) is a middle piece, and wherein the catheter comprises a second connection tube (2, 4), wherein the middle piece (3) has a distal fastening piece (13) for fastening to the first connection tube (2, 4) and a proximal fastening piece (14) for fastening to the second connection tube (2, 4).

5. The catheter according to one of Claim 1 to 4, wherein the catheter further comprises a rigid head part (1), wherein the head part (1) has a third fastening piece (9) for fastening to the first connection tube (2, 4), such that the head part (1) is arranged at a distance from the stiff element (3).

6. The catheter according to one of Claims 1 to 5, wherein at least the first connection tube (2, 4) is flexible.

7. The catheter according to one of Claims 1 to 6, wherein the stiff element (3) is made of metal or plastic.

8. The catheter according to one of Claims 1 to 7, wherein the first and the second window (300, 301) are arranged on the same side of the stiff element (3) .

9. The catheter according to one of Claims 2 or 3, wherein the lower part (31) is transparent for visible light and/or for UV light.

10. The catheter according to one of Claims 1 to 9, wherein a temperature sensor (26) is arranged in the stiff element (3) between the first and the second window (300, 301).

11. The catheter according to Claim 10, wherein the temperature sensor (26) is a diode.

12. The catheter according to Claim 4, wherein the second connection tube (4) comprises an inner tube (4') having a distal end and an outer tube (4") having a distal end, wherein the distal end of the outer tube (4'') protrudes the distal end of the inner tube (4') and wherein the second tube (4") is attached to the proximal fastening piece (14) with at least the outer tube (4").

13. The catheter according to Claim 12, wherein the second tube (4) is attached to the proximal fastening piece (14) with the outer tube (4") only.

14. The catheter according to one of Claims 12 or 13, wherein the inner tube (4') is bonded to the outer tube (4").

15. The catheter according to one of Claims 1 to 14, wherein the light emitter is an optical conductor that has a distal end, and wherein the distal end is held in the stiff element (3).

16. The catheter according to one of Claims 1 to 15, wherein a pressure sensor (27) and/or a temperature sensor (26) are arranged in the stiff element (3) or in a tip element (1).

17. The catheter according to one of Claims 1 to 16, wherein the at least one light receiver is a photodetector (25) arranged in the stiff element (3) .

18. The catheter according to Claim 17, wherein a receiver in which a circuit board (24) is secured is present in the recess (16, 16'), and wherein at least the photodetector (25) is arranged on the circuit board (24).

19. The catheter according to one of Claims 1 to 18, wherein a mirror (18) is present in the stiff element (3), lies opposite a light exit surface area of the light emitter and deflects light issuing from the light emitter into the body tissue.

20. The catheter according to one of Claims 1 to 19, wherein it has a drainage channel (12, 12') extending through the stiff element (3).

21. The catheter according to one of Claims 1 to 20, wherein the catheter has a smooth and unchanging diameter at least along the first tube (2, 4) and the stiff element (3).

22. The catheter according to Claims 4 and 21, wherein the catheter has a smooth and unchanging diameter at least along the first tube (2, 4), the stiff element (3) and the second tube (2, 4).

## Patentansprüche

1. Ein zerebraler Gewebe-Katheter zur Messung einer Durchblutung durch ein zerebrales Gewebe eines Patienten,
wobei der Katheter ein ausserhalb des Patienten verbleibendes, proximales Ende und ein in den Patienten eingeführtes distales Ende aufweist,
wobei der Katheter einen ersten Verbindungsschlauch (2, 4), ein steifes Element (3) mit mindestens einem ersten Fenster (300) und einem zweiten Fenster (301) und einen Führungskanal (12, 12') aufweist,
wobei sich der Führungskanal (12, 12') vom proximalen Ende zum distalen Ende erstreckt und wobei sich der Führungskanal (12, 12') durch das steife Element (3) erstreckt, wobei der Führungskanal (12, 12') geeignet ist, einen steifen Draht aufzunehmen, wodurch der steife Draht durch den Katheter bewegbar ist,
wobei ein Lichtemitter zur Emission von Licht in das Körpergewebe und mindestens ein Lichtempfänger zum Empfang von im Körpergewebe und zurück in den Katheter reflektiertem Licht im steifen Element (3) angeordnet sind,
wobei das vom Lichtemitter emittierte Licht durch das zweite Fenster (301) in das Körpergewebe emittiert ist und das im Körpergewebe reflektierte Licht durch das erste Fenster (300) vom mindestens einen Lichtempfänger empfangen ist.

2. Der Katheter gemäss Anspruch 1, wobei das steife Element (3) einen oberen Teil (30) und einen unteren Teil (31) aufweist, die miteinander verbunden sind, wobei das mindestens eine erste und zweite Fenster (300, 301) im oberen Teil (30) angeordnet sind, wobei der obere Teil (30) für die verwendete Wellenlänge nicht durchsichtig ist.

3. Der Katheter gemäss Anspruch 2, wobei der untere Teil (31) für eine während dem Zusammenbau des unteren und oberen Teils (30, 31) verwendete Wellenlänge durchsichtig ist.

4. Der Katheter gemäss einem der Ansprüche 1 bis 3, wobei das steife Element (3) ein Mittelstück ist, und wobei der Katheter einen zweiten Verbindungsschlauch (2, 4) aufweist, wobei das Mittelstück (3) ein distales Befestigungsstück (13) zur Befestigung am ersten Verbindungsschlauch (2, 4) und ein proximales Befestigungsstück (14) zur Befestigung am zweiten Verbindungsschlauch (2, 4) aufweist.

5. Der Katheter gemäss einem der Ansprüche 1 bis 4, wobei der Katheter ferner ein steifes Kopfteil (1) aufweist, wobei das Kopfteil (1) ein drittes Befestigungsstück (9) zur Befestigung am ersten Verbindungsschlauch (2, 4) aufweist, so dass das Kopfteil (1) in einem Abstand zum steifen Element (3) angeordnet ist.

6. Der Katheter gemäss einem der Ansprüche 1 bis 5, wobei mindestens der erste Verbindungsschlauch (2, 4) flexibel ist.

7. Der Katheter gemäss einem der Ansprüche 1 bis 6, wobei das steife Element (3) aus Metall oder Kunststoff gefertigt ist.

8. Der Katheter gemäss einem der Ansprüche 1 bis 7, wobei das erste und das zweite Fenster (300, 301) auf derselben Seite des steifen Element (3) angeordnet sind.

9. Der Katheter gemäss einem der Ansprüche 2 oder 3, wobei der untere Teil (31) für sichtbares Licht und/oder für UV Licht durchsichtig ausgebildet ist.

10. Der Katheter gemäss einem der Ansprüche 1 bis 9, wobei ein Temperatursensor (26) im steifen Element (3) zwischen dem ersten und dem zweiten Fenster (300, 301) angeordnet ist.

11. Der Katheter gemäss Anspruch 10, wobei der Temperatursensor (26) eine Diode ist.

12. Der Katheter gemäss Anspruch 4, wobei der zweite Verbindungsschlauch (4) einen inneren Schlauch (4') mit einem distalen Ende und einen äusseren Schlauch (4") mit einem distalen Ende aufweist, wobei das distale Ende des äusseren Schlauchs (4") dem distalen Ende des inneren Schlauchs (4') vorsteht und wobei der zweite Schlauch (4") mindestens mit dem äusseren Schlauch (4") am proximalen Befestigungsstück (14) befestigt ist.

13. Der Katheter gemäss Anspruch 12, wobei der zweite Schlauch (4) nur mit dem äusseren Schlauch (4") am proximalen Befestigungsstück (14) befestigt ist.

14. Der Katheter gemäss einem der Ansprüche 12 oder 13, wobei der innere Schlauch (4') mit dem äusseren Schlauch (4") verbunden ist.

15. Der Katheter gemäss einem der Ansprüche 1 bis 14, wobei der Lichtemitter ein optischer Leiter ist, der ein distales Ende aufweist, und wobei das distale Ende im steifen Element (3) gehalten ist.

16. Der Katheter gemäss einem der Ansprüche 1 bis 15, wobei ein Drucksensor (27) und/oder ein Temperatursensor (26) im steifen Element (3) oder in einem Spitzenelement (1) angeordnet sind.

17. Der Katheter gemäss einem der Ansprüche 1 bis 16, wobei der mindestens eine Lichtempfänger ein Photodetektor (25) ist, der im steifen Element (3) angeordnet ist.

18. Der Katheter gemäss Anspruch 17, wobei ein Empfänger, in welchem eine Leiterplatte (24) gesichert ist, in der Ausnehmung (16, 16') vorhanden ist, und wobei mindestens der Photodetektor (25) auf der Leiterplatte (24) angeordnet ist.

19. Der Katheter gemäss einem der Ansprüche 1 bis 18, wobei ein Spiegel (18) im steifen Element (3) vorhanden ist, der einer Lichtauslassoberfläche des Lichtemitters gegenüberliegt und der vom Lichtemitter in das Körpergewebe ausgestrahlte Licht umlenkt.

20. Der Katheter gemäss einem der Ansprüche 1 bis 19, wobei er einen Drainagekanale (12, 12') aufweist, der sich durch das steife Element (3) erstreckt.

21. Der Katheter gemäss einem der Ansprüche 1 bis 20, wobei der Katheter mindestens entlang des ersten Schlauchs (2, 4) und dem steifen Element (3) einen glatten und sich nicht verändernden Durchmesser aufweist.

22. Der Katheter gemäss den Ansprüchen 4 und 21, wobei der Katheter entlang des ersten Schlauchs (2, 4), dem steifen Element (3) und dem zweiten Schlauch (2, 4) einen glatten und sich nicht verändernden Durchmesser aufweist.

## Revendications

1. Cathéter de tissu cérébral pour mesurer un flux sanguin à travers un tissu cérébral d'un patient,
le cathéter possédant une extrémité proximale restant à l'extérieur du patient et une extrémité distale devant être introduite dans le patient,
le cathéter comprenant un premier tube de raccordement flexible (2, 4), un élément rigide (3) comprenant au moins une première fenêtre (300) et une seconde fenêtre (301) et un canal de guidage (12, 12'), le canal de guidage (12, 12') s'étendant depuis l'extrémité proximale vers l'extrémité distale et le canal de guidage (12, 12') s'étendant à travers l'élément rigide (3), le canal de guidage (12, 12') pouvant recevoir un fil rigide, ce qui permet que le fil rigide soit déplacé à travers le cathéter,
dans lequel un émetteur de lumière pour émettre de la lumière vers le tissu corporel et au moins un récepteur de lumière pour recevoir la lumière réfléchie dans le tissu corporel et renvoyée au cathéter sont disposés dans l'élément rigide (3), dans lequel la lumière émise depuis l'émetteur de lumière est émise à travers la seconde fenêtre (301) vers le tissu corporel et la lumière réfléchie dans le tissu corporel est reçue par l'au moins un récepteur de lumière à travers la première fenêtre (300).

2. Cathéter selon la revendication 1, dans lequel l'élément rigide (3) comprend une partie supérieure (30) et une partie inférieure (31) étant raccordées entre elles, dans lequel les au moins première et seconde fenêtres (300, 301) sont disposées dans la partie supérieure (30), dans lequel la partie supérieure (30) n'est pas transparente pour la longueur d'onde utilisée.

3. Cathéter selon la revendication 2, dans lequel la partie inférieure (31) est transparente au moins pour une longueur d'onde utilisée pendant l'assemblage des parties inférieure et supérieure (30, 31).

4. Cathéter selon l'une des revendications 1 à 3, dans lequel l'élément rigide (3) est une pièce médiane, et dans lequel le cathéter comprend un second tube de raccordement (2, 4), dans lequel la pièce médiane (3) possède une pièce de fixation distale (13) pour une fixation au premier tube de raccordement (2, 4) et une pièce de fixation proximale (14) pour une fixation au second tube de raccordement (2, 4).

5. Cathéter selon l'une des revendications 1 à 4, dans lequel le cathéter comprend en outre une partie tête rigide (1), dans lequel la partie tête (1) possède une troisième pièce de fixation (9) pour une fixation au premier tube de raccordement (2, 4), de sorte que la partie tête (1) soit disposée à une certaine distance de l'élément rigide (3).

6. Cathéter selon l'une des revendications 1 à 5, dans lequel au moins le premier tube de raccordement (2, 4) est flexible.

7. Cathéter selon l'une des revendications 1 à 6, dans lequel l'élément rigide (3) est en métal ou en plastique.

8. Cathéter selon l'une des revendications 1 à 7, dans lequel les première et seconde fenêtres (300, 301) sont disposées sur le même côté de l'élément rigide (3).

9. Cathéter selon l'une des revendications 2 et 3, dans lequel la partie inférieure (31) est transparente pour la lumière visible et/ou pour la lumière ultraviolette.

10. Cathéter selon l'une des revendications 1 à 9, dans lequel un capteur de température (26) est disposé dans l'élément rigide (3) entre les première et seconde fenêtres (300, 301).

11. Cathéter selon la revendication 10, dans lequel le capteur de température (26) est une diode.

12. Cathéter selon la revendication 4, dans lequel le second tube de raccordement (4) comprend un tube interne (4') possédant une extrémité distale et un tube externe (4") possédant une extrémité distale, dans lequel l'extrémité distale du tube externe (4") dépasse de l'extrémité distale du tube interne (4') et dans lequel le second tube (4") est fixé à la pièce de fixation proximale (14) avec au moins le tube externe (4").

13. Cathéter selon la revendication 12, dans lequel le second tube (4) est fixé à la pièce de fixation proximale (14) avec le tube externe (4") uniquement.

14. Cathéter selon l'une des revendications 12 et 13, dans lequel le tube interne (4') est lié au tube externe (4").

15. Cathéter selon l'une des revendications 1 à 14, dans lequel l'émetteur de lumière est un conducteur optique qui possède une extrémité distale, et dans lequel l'extrémité distale est tenue dans l'élément rigide (3).

16. Cathéter selon l'une des revendications 1 à 15, dans lequel un capteur de pression (27) et/ou un capteur de température (26) sont disposés dans l'élément rigide (3) ou dans un élément de pointe (1).

17. Cathéter selon l'une des revendications 1 à 16, dans lequel l'au moins un récepteur de lumière est un photodétecteur (25) disposé dans l'élément rigide (3).

18. Cathéter selon la revendication 17, dans lequel un récepteur dans lequel est fixée une carte de circuit imprimé (24) est présent dans le renfoncement (16, 16'), et dans lequel au moins le photodétecteur (25) est disposé sur la carte de circuit imprimé (24).

19. Cathéter selon l'une des revendications 1 à 18, dans lequel un miroir (18) est présent dans l'élément rigide (3), est situé en regard d'une surface de sortie de lumière de l'émetteur de lumière et dévie la lumière sortant de l'émetteur de lumière vers le tissu corporel.

20. Cathéter selon l'une des revendications 1 à 19, dans lequel se trouve un canal de drainage (12, 12') s'étendant à travers l'élément rigide (3).

21. Cathéter selon l'une des revendications 1 à 20, le cathéter ayant un diamètre uniforme et immuable au moins le long du premier tube (2, 4) et de l'élément rigide (3).

22. Cathéter selon les revendications 4 et 21, le cathéter ayant un diamètre uniforme et immuable au moins le long du premier tube (2, 4), de l'élément rigide (3) et du second tube (2, 4).
